# EUROPEAN PATENT APPLICATION

(11) **EP 0 856 332 A1**
(43) Date of publication of application: **05.08.1998**
(21) Application number: 98300705.5
(22) Date of filing: 30.01.1998
(51) Int. Cl.: A61M 39/12, F16L 33/00

(54) **Adapter for mounting a fluid handling device on a catheter tubing**

(30) Priority: 31.01.1997 US 792640
(71) Applicant: Becton, Dickinson and Company, Franklin Lakes, New Jersey 07417-1880 (US)
(72) Inventor: Daw, Sean P., Brooklyn, New York 11231 (US); Houghton, Frederick C., Sussex, New Jersey 07461 (US)
(74) Representative: Ruffles, Graham Keith

(57) **Abstract**

An adapter for attaching a fluid handling device to a catheter tubing of the present invention includes a body with a proximal end, a distal end and a passageway therethrough that defines a longitudinal axis. The proximal end of the adapter includes a fitting for attaching a fluid handling device and the distal end of the body includes a receptacle to accept and to form a releasable substantially fluid-tight connection to a preselected catheter tube that has an outside diameter. The adapter of the invention has a latch member disposed over the receptacle. The latch member has an axial access port therein with an inside surface to allow placement of a proximal end of a catheter tube into the receptacle. The latch member is disposed for rotatable movement with respect to the body between an insertion position wherein the receptacle is accessible to the catheter and a latched position to retain the catheter in the adapter. The adapter of the invention is constructed so that it is visually and tactily indicative of the position of the latch member with respect to the body, thereby enabling the practitioner to easily discern whether the adapter is in the insertion position or the latched position.

## Description

Field of Invention: The present invention is generally related to the field of catheters and more particularly to adapters for mounting a fluid handling device on a catheter tubing.

### Background:

Catheters are elongate hollow tubes that are used to transmit fluids into or out of the body of a patient. Conventions followed for the devices described in this disclosure are that the term "proximal" is the direction away from the patient and toward the practitioner and the term "distal" refers to the direction toward the patient and away from the practitioner. There are many types of catheters currently used in medical practice. Some catheters are sufficiently strong and rigid to be introduced by themselves, urinany catheters are examples of this type of catheter. Another catheter type is positioned on the outside of a sharp introducer needle and slid down over the needle into the patient's body using the needle to make the penetration and provide a guide to placement of the catheter, many intravenous catheters are of this type. This disclosure is related to yet another type, a catheter that is introduced into the patient through the bore of a sharp introducer needle. Through-the-needle catheters are further separated into two types by the introducer needle. When a through-the-needle catheter has a fixed hub for attachment of a fluid handling device, the introducer needle cannot be slid off the proximal end of the catheter. Catheters with fixed hubs either are used with a splittable introducer needle or the needle must be left on the catheter. One important application of catheters in medical practice is the use of long flexible catheters to introduce medicaments, often anesthetic or analgesic formulations, into the spine of a patient. In this application, the long (50-75 cm) flexible catheter tubing ( generally 19-21 gauge) is introduced into the patient's epidural space through the bore of an introducer needle.

These spinal anesthesia procedures are widely used in hospital practice, with the generic name of "an epidural." As an example, the use of an epidural anesthetic is described in obstetric practice. The epidural anesthetic procedure is useful in many other type of procedures. In a typical obstetric procedure, the epidural catheter is often placed early in the patient's labor with the patient lying on her side then the patient is placed on her back with the knees elevated for the rest of the delivery. Since the patient is on her back, the introducer needle generally must be removed. Most epidural catheters do not have fixed hubs thus allowing the introducer needle to be slid proximally off of the catheter and removed. Once the needle is removed, it is necessary to mount an adapter onto the catheter so that a fluid handling device such as a syringe may be attached to the catheter. The adapter is then often secured with tape onto the patient's body. The Tuohy-Borst adapter was developed for this application. The Tuohy-Borst adapter allows a fluid handling device with a male luer fitting to be mounted onto a small diameter (generally 19-21 gauge: Nominal Outside Diameters for these 19 to 21 gauges are between about 1.10mm [19 gauge] to about 0.8mm [21 gauge] ) flexible catheter tube. The original Tuohy-Borst adapter is formed from metal and is considered reusable. Other variants of the original Tuohy-Borst are now available formed from thermoplastics. The thermoplastic adapters are generally supplied sterile and are considered single-use and disposable. The Tuohy-Borst type adapters all depend in some degree on a threaded collar being screwed down around the catheter to compress a resilient plug contained in a body portion. The seal around the catheter is formed by compressing the tip of the resilient plug into a cavity around the catheter tube by screwing the collar down onto the plug. In most of these adapters, it is easy for a practitioner to inadvertently over-tighten the threaded collar and occlude the catheter lumen. Alternatively, if the collar is not tightened down sufficiently, the adapter may leak or may even come off of the catheter tube. Most of the available adapters are generally cylindrical, may include a releasable latch mechanism and require at least about one-half rotation of the collar portion with respect to the body portion to secure the adapter onto the catheter.

A widely used adapter, available from B. Braun, Bethlehem, PA, has a collar and a body portion. The Braun adapter is capable of almost four complete rotations of the collar with respect to the body portion from the initial engagement of the threads. Additionally, if this collar of the B. Braun adapter is fully unthreaded from the body portion, it may detach and allow disassembly of the adapter. Another widely used adapter is the disposable successor to the reusable Tuohy-Borst available from Becton Dickinson and Company, Franklin Lakes, NJ. The collar of this successor adapter is fully seated on the body after only about two and one half rotations of the collar with respect to the body. Additionally, unlike the B. Braun adapter, the collar is retained on the body when completely unthreaded so that it cannot easily fall off. Another available adapter, as disclosed in US Patent Nos 5,053,015 and 5,226,898, has an external ratchet and includes small wings on both the body and the collar to facilitate the practitioners handling and, when the wings are aligned, provides some indication that the adapter is secured onto the tubing. When the adapter disclosed in the referenced patents is secured to the patient's body, the small wings may cause discomfort to the patient, and additionally, the adapter may sometimes be difficult for a gloved practitioner to handle.

Other than the catheters disclosed in U.S. Patent Nos. 5,053,015 and 5,226,898, substantially all of the available adapters do not provide the practitioner with much indication of the sufficiency of the degree of tightness of the collar with respect to the body, and it is not easily visually apparent if the collars are loosened so that the catheter tubing may be inserted or if the collar is partially screwed down on the body, making it difficult to insert the catheter tube into the body. Operating room time is expensive, and, additionally, many procedures are conducted under time constraints that potentially have impact on the patient's well being. As a result, practitioners and their support staff make every effort to set up repeatable procedures with standardized placements of equipment to facilitate rapid implementation of procedures. If a practitioner attempts to put an adapter onto a catheter tube and has difficulty because the adapter is partially threaded, additional time is required. If a practitioner inadvertently over-tightens a collar of an adapter occluding the lumen, he may believe the catheter is clogged, remove it and have to repeat the placement. The repeat procedure not only subjects the patient to additional risk, but also significantly increases the time required. If an adapter is not sufficiently tightened, it may fall off or leak during an extended procedure, thereby resulting in improper patient medication. If a gloved practitioner has difficulty handling an adapter and drops it, there may be a time delay while another adapter is procured, and, in the case where the adapter is part of a procedure kit, another whole kit, with a significant cost increment, may need to be opened just to obtain another adapter.

If a catheter adapter that was easily handled by a gloved practitioner was available that provided the practitioner with a positive indication of its status, i. e., ready-to-receive a catheter tube or fully tightened, that could not be over-tightened, required only about one quarter turn of the collar with respect to the body to be fully tightened, and where the practitioner could readily ascertain if the catheter was properly positioned in the adapter before tightened, the art of catheter adapters would be advanced. Such a catheter adapter is disclosed below.

### Summary of the Invention:

An adapter of the present invention for attaching a fluid handling device to a catheter tubing includes a body with a proximal end, a distal end and a passageway therethrough that defines a longitudinal axis. The proximal end of the adapter includes a fitting for attaching a fluid handling device and the distal end of the body includes a receptacle to accept and to form a releasable, substantially fluid-tight connection to a preselected catheter tube that has an outside diameter. The adapter of the invention has a latch member disposed over the receptacle. The latch member has an axial access port therein with an inside surface to allow placement of a proximal end of a catheter tube into the receptacle. The latch member is disposed for rotatable movement with respect to the body between an insertion position wherein the receptacle is accessible to the catheter and a latched position to retain the catheter in the adapter. The adapter of the invention is constructed so that it is visually and tactily indicative of the position of the latch member with respect to the body, thereby enabling the practitioner to easily discern whether the adapter is in the insertion position and ready to receive the catheter and when the adapter is latched to retain the catheter.

Catheters intended for placement through the bore of an introducer needle are widely used in treatments requiring fluid transmission into the patients' spinal column. The introducer needle is generally removed after the catheter is positioned and the treatment is begun. Generally, when conventional needles are used for the insertion and after the catheter is properly positioned, the introducer needle is withdrawn from the patient, slid back to the proximal end of the catheter and removed. Although some splittable needles are in use, conventional introducer needles are generally preferred by practitioners. When the conventional needles are used, the catheter cannot have a proximal fixed bonded hub, because the needle must pass over the proximal end of the catheter. Many adapters have been developed to attach fluid handling devices to the catheters. The catheter adapter of the present invention provides practitioners with an easily attached adapter that is more easily correctly positioned on the catheter and securely latched to retain the adapter on the catheter than previously disclosed adapters.

### Brief Description of the Drawings:

Fig. 1 is an exploded skeletonized perspective view of the adapter of the present invention with the latch member in the insertion position;
Fig. 2 is a skeletonized perspective view of the invention of Fig. 1 with the latch member in the insertion position and a catheter tubing partially positioned;
Fig. 3 is a perspective view analogous to Fig. 2 with the catheter tubing fully positioned;
Fig. 4 is a skeletonized perspective view of the of the invention of Fig. 1 with the catheter tubing fully positioned and the adapter latched.
Fig. 5 is a distal end view of the invention of Fig. 1;
Fig. 6 is a top plan view of the invention of Fig. 1, analogous to Fig. 3, with the catheter tubing fully positioned and the latch member in the unlatched position relative to the body;
Fig. 7 is a skeletonized perspective view of the invention of Fig. 1, analogous to Fig. 4, with the catheter tubing fully positioned;
Fig. 8 is a cross-sectional view of the adapter of Fig. 1 taken along the line 8-8;
Fig. 9 is a cross-sectional view taken from Fig. 3 along the line 9-9;
Fig. 10 is a cross-sectional view taken from Fig. 4 along the line 10-10;
Fig. 11 is a view of the distal portion of the adapter from Fig. 8;
Fig. 12 is a view of the distal portion of the adapter from Fig. 9; and
Fig. 13 is a skeletonized perspective view of the resilient member of the adapter of the invention as shown in Fig. 1.

### Detailed Description:

While this invention is satisfied by embodiments in many different forms, there are shown in the drawings and herein described in detail, embodiments of the invention with the understanding that the present disclosure is to be considered as exemplary of the principles of the present invention and is not intended to limit the scope of the invention to the embodiments illustrated. The scope of the invention is measured by the appended claims and their equivalents.

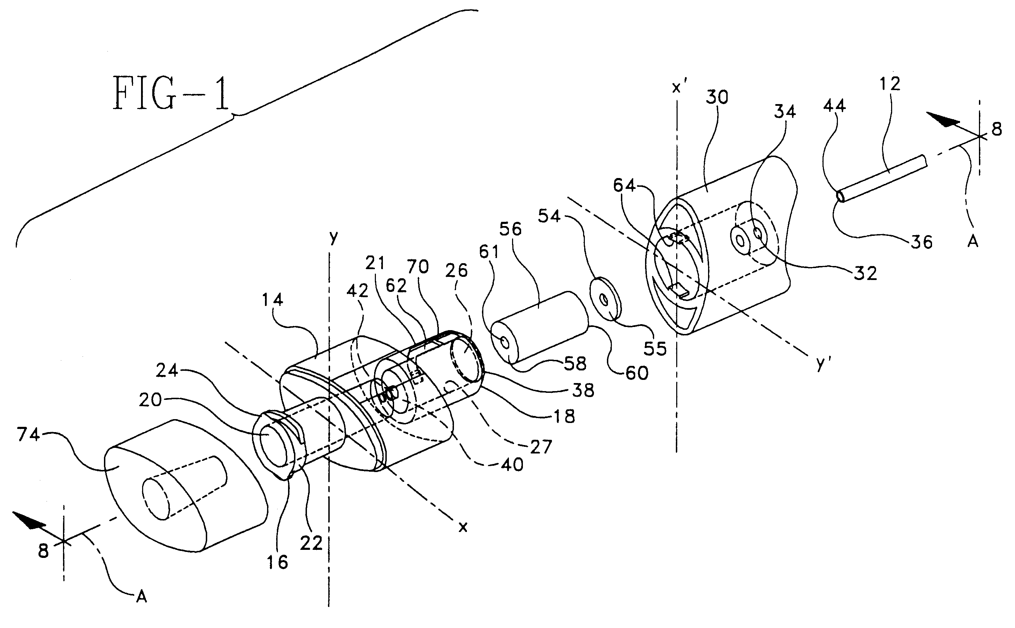

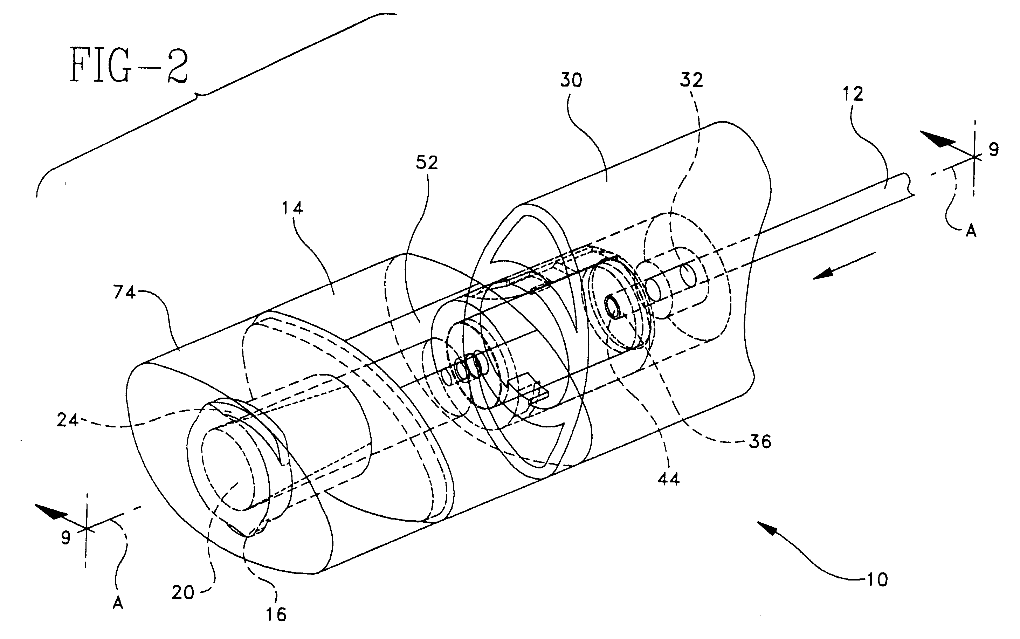

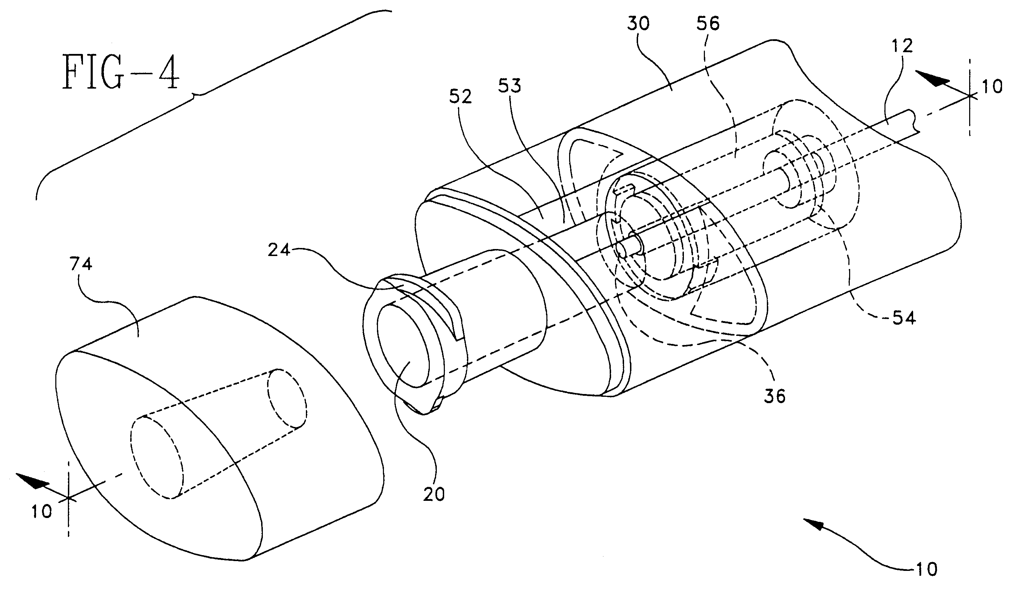

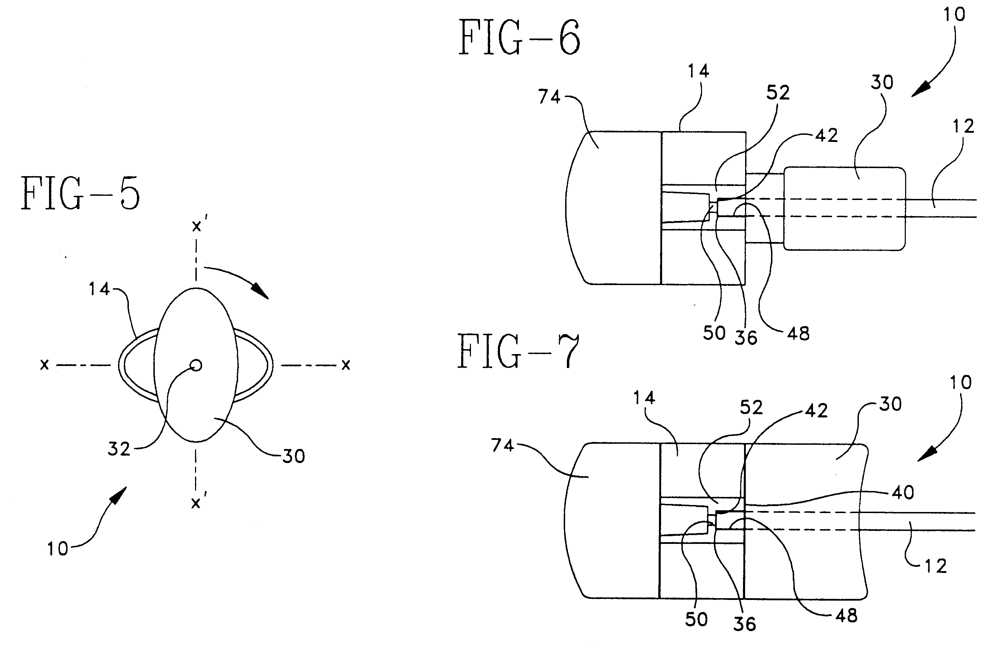

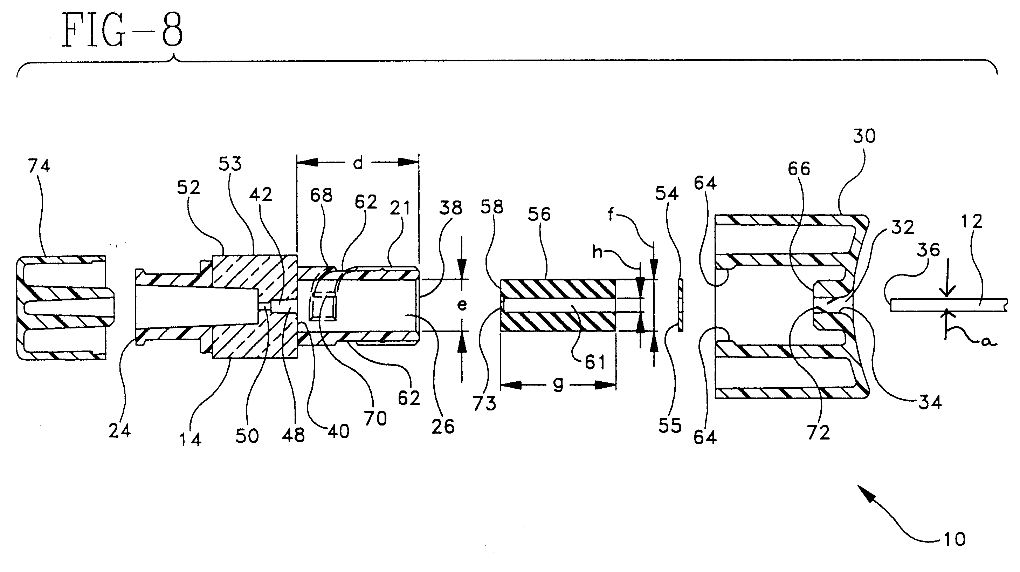

Referring to Figs 1-13, an adapter 10 of the present invention for attaching a fluid handling device to a catheter 12 includes a body 14 with a proximal end 16, a distal end 18 and a passageway 20 therethrough defining a longitudinal axis A and an outside surface 21. Proximal end 16 includes a fitting 22, preferably a female luer fitting 24, to attach a fluid handling device. Distal end 18 of body 14 includes a receptacle 26 with an interior surface 27 to accept, and to form a releasable substantially fluid tight connection to preselected catheter tube 12 with an outside diameter "a". Adapter 10 has a latch member 30 disposed over receptacle 26. Latch member 30 has an axial access port 32 therein with an inside surface 34 to receive a proximal end 36 of catheter tube 12 for placement into receptacle 26, latch member 30 is disposed for rotatable movement with to respect to body 14 between an insertion position, best seen in Figs 1, 2 and 6, where receptacle 26 is accessible to catheter 12 and a latched position, best seen in Figs. 4 and 7 to retain catheter 12 in receptacle 26. As shown in Fig. 1, latch 30 and body 14 are preferably each formed in shape of an elongate tube with substantially identical elliptical cross-sections, each shape having a long axis "x, x' ", respectively, and a short axis "y,y' ", respectively, and disposed so that when latch 30 is in the latched position with respect to body 14, as seen in Fig. 4, long axes x,x' and short axes y,y' are substantially aligned and when latch 30 is in the insertion position as seen in Fig. 1, short axis y of body 14 is substantially aligned with long axis x' of latch 30 with long axis x of the body being substantially aligned with short axis y' of latch 30, thereby providing a visual and tactile indication of the position of latch 30 with respect to body 14 and leverage to facilitate the practitioner's rotatable movement of latch 30 with respect to body 14. Many other shapes having asymmetric cross-sections, in addition to the preferred elliptical shape, for adapter 10 may be envisioned and are considered to be within the scope of the invention. An additional benefit of the preferred elongate elliptical shape for the adapter of the invention is that the preferred shape is less likely than the currently available adapters to cause discomfort to the patient by being pressed into the patient's flesh while providing an enlarged surface area for the practitioner to apply tape for securing the adapter to the patient. Adapter 10 of the invention is substantially easier for a gloved practitioner to handle than most currently available adapters because of the preferred elongate eliptical shape.

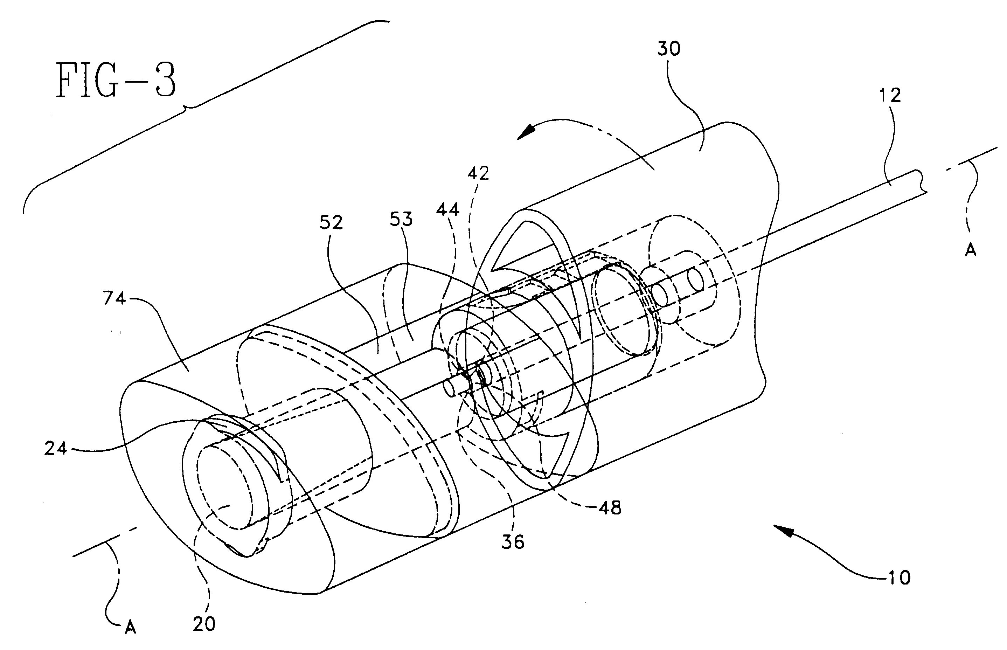

Receptacle 26 is generally coaxial with passageway 20 with an open distal end 38 and has a closed bottom 40 defining a catheter seat 42 for fluidly connecting a lumen 44 of catheter 12 to proximal fitting 24. Receptacle 26 has a depth "d" and an inside diameter "e". Catheter seat 42 includes an axial recess 48 therein that has an axial opening 50 in closed bottom 40 of receptacle 26. Recess 48 is sized to receive proximal end 36 of the catheter. Receptacle 26 includes a generally cylindrical resilient member 56 that is disposed substantially coaxially within the receptacle. Resilient member 56 has a proximal end 58, a distal end 60 and has an axial bore 61 therethrough. Resilient member 56 has an outside diameter "f" sized to fit within receptacle 26 and a length "g" substantially equal to depth "d" of receptacle 26. Axial bore 61 of resilient member 56 has an inside diameter "h" that is larger than outside diameter "a" of preselected catheter 12. While the outside surface of the body and the latch of the adapter may have a textured surface to facilitate handling by a gloved practitioner, preferably, as seen in Figs. 3, 4, 6 and 7, at least a portion of body 14 has a substantially transparent viewing area 52 so that when the proximal end 36 of the catheter is properly positioned in recess 48 of catheter seat 42 with lumen 44 of the catheter substantially aligned with opening 50, proximal end 36 of the catheter is visible to the practitioner through area 52. Additionally, transparent viewing area 52 is preferably formed into a shape 53, such as a cylinder lens or any other shape to provide an enlarged image of recess 48, thereby enhancing the practitioner's visualization of proper placement of the catheter.

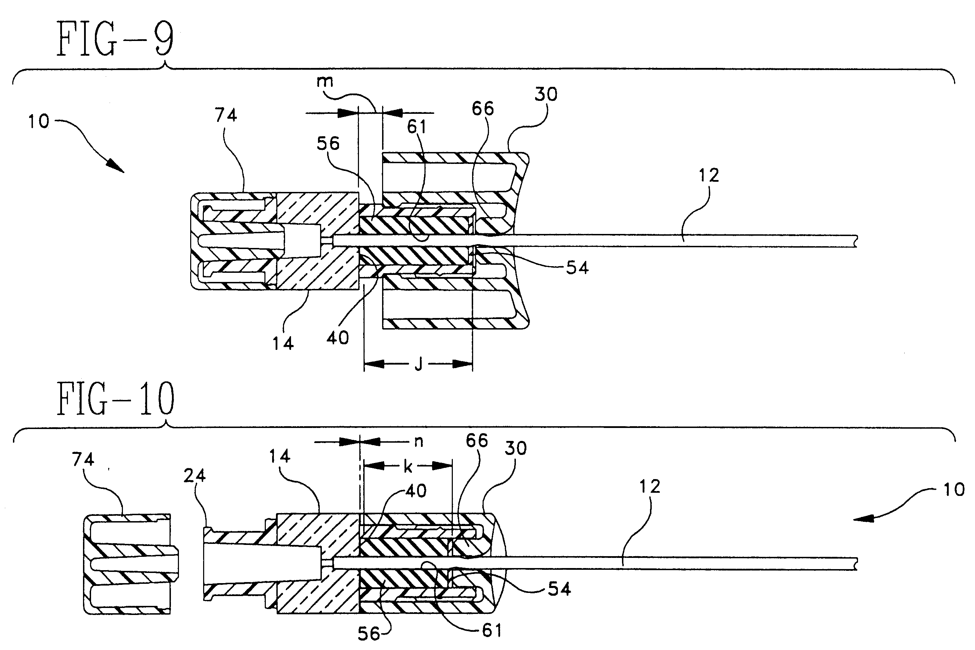

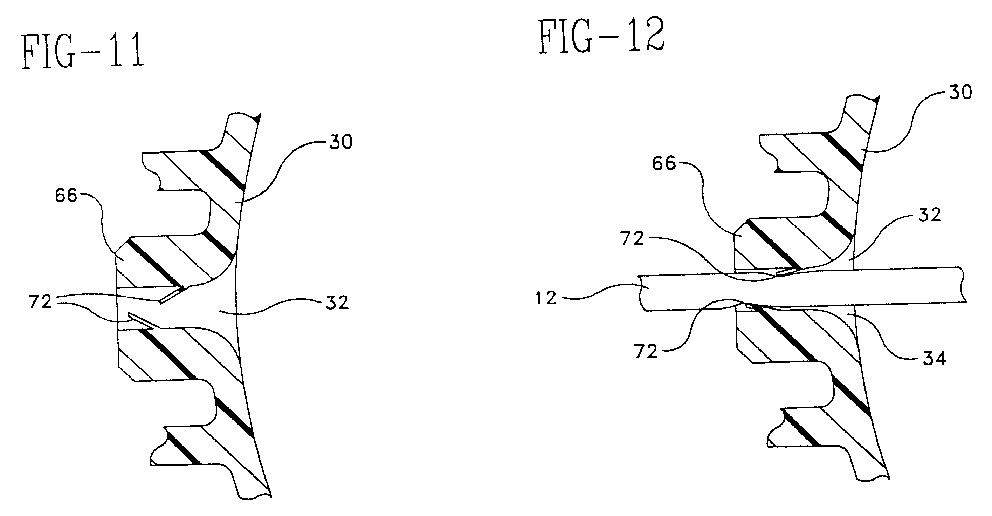

As seen in Fig. 8, outside surface 21 of body 14 includes at least one, preferably two recessed, camming surfaces 62 and latch member 30 includes at least one, preferably two projecting, cam followers 64 disposed to engage camming surfaces 62. Referring to Fig. 8, latch member 30 also has a compression member 66 disposed to engage resilient member 56. Preferably receptacle 26 further includes a washer 54 disposed proximally to compression member 66 with resilient member 56 proximal to washer 54. When latch member 30 is rotated from the insertion position, shown in Fig. 9, to the latched position, shown in Fig. 10, with the preselected catheter 12 positioned on catheter seat 42, latch member 30 is advanced proximally from a distance "m" distal to body 14 to a distance "n" distal to body 14. Preferably, when latch 30 is in the latched position, illustrated in Fig. 10, latch member 30 is substantially in contact with body 14, i.e., distance "n" is substantially zero. The movement of latch 30 from distal distance "m" to distance "n" results in compression member 66 applying a compressive force against washer 54 to compress resilient member 56 against closed bottom 40 and inside surface 27 of receptacle 26 and thereby reduce inside diameter "h" of axial bore 61. When inside diameter h of the resilient member is reduced to less than outside diameter "a" of the preselected catheter an interference fit retains catheter 12 in adapter 10. Washer 54 provides a surface 55 for distal end 60 of the resilient member to be compressed against and allows for rotation of latch member 30 with respect to the resilient member during compression to substantially reduce any tendency of the resilient member twist and to occlude the catheter lumen. Additionally, a profile 68 of camming surfaces 62 is selected so that the rotatable movement of latch 30 with respect to body 14 is less than about one half turn and, preferably, only about one quarter turn. The preferred limitation of one quarter turn to complete the movement between the latched and the unlatched position makes over-tightening with concomitant occlusion of the catheter lumen substantially less likely than current adapters with a threaded fitting or ratcheted fitting that requires an indeterminate amount of rotation of the collar with respect to the body to achieve catheter retention. Additionally, with the adapter of the invention, it is readily apparent to the practitioner whether the adapter is latched or unlatched. Most current adapters do not provide an indication if they are sufficiently tightened or not, and, if the adapter is not tightened sufficiently, the catheter may leak or become dislodged from the adapter.

Camming surfaces 62 further preferably include at least one protuberance 70 that is sized and positioned to releasably retain cam followers 64 when latch 30 is moved to the latched position, thus retaining adapter 10 on catheter 12 until the practitioner desires its release. Preferably, protuberances 70 are sized and shaped so as to produce a "snap" when latch 30 is moved between the unlatched and the latched position to provide the practitioner with a positive aural and tactile indication that the movement from one position to another is complete.

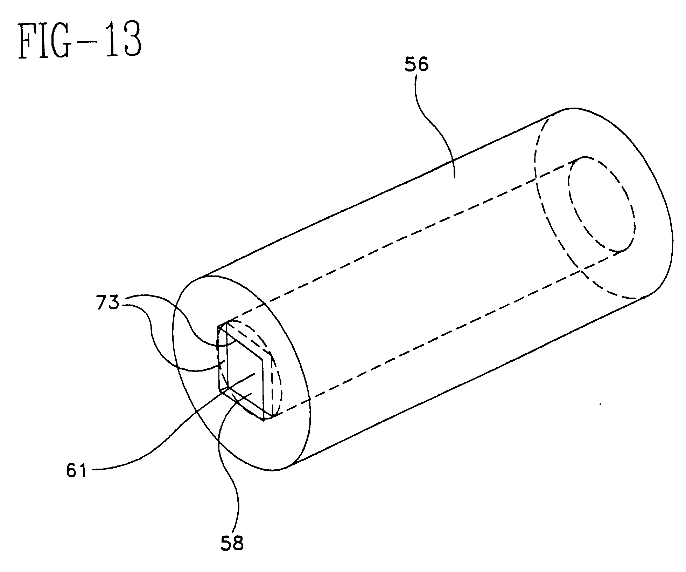

Preferably, axial access port 32 provides that a force for inserting preselected catheter tube 12 into adapter 10 is less than a force for withdrawal of catheter tube 12 from the adapter before latch 30 is moved with respect to body 14, thereby substantially reducing inadvertent catheter tube withdrawal from the adapter prior to latching. Preferably this differential withdrawal force of the catheter is provided by having at least one inward projection 72 on inside surface 34 of axial access port 32. Projection 72 is preferably disposed to facilitate proximal motion of catheter tube 12 and to provide a resistance to distal motion of catheter tube 12. Referring to Figs. 11 and 12, inside surface 34 of access port 32 preferably has two inward projections.

Referring to Fig. 13, resilient member 56 is preferably provided with flexible flaps 73 that partially obstruct the axial bore 61 at proximal end 58. Flexible flaps 73 result from the gate used to fill a cavity for forming resilient member 56 when an injection molding process is selected for the formation. Flexible flaps 73 deflect as the catheter is inserted and contribute to the retention of catheter 12 in the adapter prior to latching. In this disclosure, flexible flaps 73 are illustrated proximally in the adapter. The retention function of flaps 73 is also seen when the orientation of resilient member results in the flaps being positioned distally, thus substantially eliminating the need to proximally or distally orient the placement of the resilient member during assembly of adapter 10.

Generally, epidural catheters are sized between about 19 gauge to about 21 gauge (outside diameter between about 1.1 mm to about 0.8 mm) and the adapter of the invention is preferably sized to accept these sizes. Other sizes of catheter tubing may be required for other applications from between about 16 gauge to about 30 gauge (outside diameter between about 1.65 mm to about 0.3 mm) and adapter 10 may be sized to accept these sizes or to accept other sizes for any other particular application.

As shown in the Figs. 1-4, adapter 10 is preferably supplied with a cap 74 over luer fitting 24. Preferably cap 74 is formed with substantially the same cross-sectional shape that is selected for the adapter to facilitate handling by the practitioner.

Body 14, cap 74 and latch member 30 may be formed from thermoplastics such as polypropylene, polycarbonate, polyamide, polyester, polyethylene, acrylonitrile/butadiene/styrene (ABS) and the like, but preferably, at least body 14 is formed from a substantially transparent material such as polycarbonate and the like to ensure that the proximal end of the catheter is readily visible through viewing area 52. Resilient member 56 may be formed from either thermoform or thermoset elastomeric materials including, but not limited to natural rubber, ethylene propylene dimer rubber (EPDM), styrene butadiene rubber (SBR), silastic rubber and the like that have a Shore A durometer preferably between about 30 and 80. Preferably resilient member 56 is formed from a latex free natural rubber. Washer 54 may be formed from a metallic material, stainless steel or the like, or other material with a low coefficient of sliding friction with either the body or the resilient member. Polycarbonate is preferred as a material for forming washer 54. All of the materials selected to form adapter 10 should be resistant to and compatible with body fluids and medications. Additionally, when material selection is made for body 14 and latch member 30, the coefficient of sliding friction between the body and latch materials should be considered so that latch 30 is readily rotatable between the latched and the unlatched position. Additionally, since preferably, the adapter of the invention is generally supplied pre-sterilized to the practitioner, either as a separate item in a package or as a component in a procedure kit, the materials selected for adapter 10 should be compatible with the particular sterilization conditions selected.

The adapter of the invention provides a practitioner with an easy-to-use adapter that is readily fitted onto a catheter tube. Proper placement of the tube on the catheter seat is readily apparent to the practitioner through the viewing area and, because of the projections in the access port and the flaps in the resilient member, the catheter tube is less likely to fall out of the adapter of the invention before if is latched. Further, the invention provides the practitioner with positive indications that the adapter is ready to receive the catheter, when the catheter is properly positioned in the adapter and when the adapter is fully engaged to retain the catheter. The preferred elongate elliptical shape of the adapter of the invention is more easily handled by a gloved practitioner than the mostly cylindrical adapters currently available.

## Claims

1. All adapter for attaching a fluid handling device to a catheter tubing comprising:
a body with a proximal end, a distal end and a passageway therethrough defining a longitudinal axis, said proximal end comprising a fitting for attaching a fluid handling device, said distal end of said body comprising a receptacle to accept and to form a releasable substantially fluid tight connection to a preselected catheter tube with an outside diameter;
a latch member disposed over said receptacle, said latch member having an axial access port therein with an inside surface for fitment of a proximal end of the catheter tube into said receptacle, said latch member being disposed for rotatable movement with respect to said body between an insertion position wherein said receptacle is accessible to the catheter and a latched position to retain the catheter in the receptacle; and
wherein said latch and said body include indicating means for indicating position of said latch with respect to said body.

2. The adapter of claim 1 wherein said receptacle is generally coaxial with said passageway, open distally with a closed bottom defining a catheter seat for connecting a lumen of the catheter to said proximal fitting, said receptacle having a depth, an inside diameter and an outside surface.

3. The adapter of claim 2 wherein said catheter seat comprises an axial recess with an axial opening through said closed bottom of said receptacle, said recess sized to receive the proximal end of the catheter, said body comprising a substantially transparent viewing area so that when the proximal end of the catheter is positioned in said recess of said catheter seat and the lumen of the catheter is substantially aligned with said opening, the proximal end of the catheter is visible.

4. The adapter of claim 3 wherein said substantially transparent viewing area of said body is formed into a shape to provide an enlarged image of said recess, thereby enhancing the practitioner's ability to properly place the catheter.

5. The adapter of claim 1 wherein said receptacle further comprises a washer and a generally cylindrical resilient member proximal to said washer, said resilient member including a proximal end, a distal end and having an axial bore therethrough, said resilient member having an outside diameter sized to fit within said receptacle and a length substantially dual to said depth of said receptacle, said axial bore of said resilient member having an inside diameter larger than the outside diameter of the preselected catheter tube.

6. The adapter of claim 5 wherein said receptacle further comprises an outside surface having at least one camming surface, said latch member including at least one cam follower disposed to engage said camming surface and a compression member disposed to engage said washer, so that when said latch member is rotated from said insertion position to said latched position with the preselected catheter positioned on said catheter seat, said latch member is advanced proximally so that a compressive force is applied to said resilient member between said washer and said catheter seat and thereby reduces said inside diameter of said axial bore to less than said outside diameter of the preselected catheter to retain the catheter in the adapter, and wherein a profile of said camming surface is selected so that said rotatable movement of said latch with respect to said body is less than about one half turn.

7. The adapter of claim 6 wherein said camming surface further includes a protuberance sized and positioned to releasably retain said cam follower when said latch is moved to said latched position.

8. The adapter of claim 1 wherein said axial access port further includes means for providing that a force for inserting the preselected catheter tube into said adapter is less than a force for withdrawal of the catheter tube from the adapter before said latch is moved with respect to said body thereby substantially reducing inadvertent catheter tube withdrawal from said adapter prior to latching.

9. The adapter of claim 1 wherein indicating means for indicating said position of said latch with respect to said body comprises said body and said latch each being formed in substantially similar asymmetric shapes so that when said latch is rotated to said latched position with respect to said body, said shapes are substantially aligned and when said latch is in said insertion position, said shapes are not substantially aligned thereby providing a visual and tactile indication of said position of said latch with respect to said body.

10. The adapter of claim 9 wherein said asymmetric shape comprises an elongate tube having substantially identical elliptical cross-sections, each shape having a long axis and a short axis, and disposed so that when said latch is in said latched position said long axes and said short axes are substantially aligned and when said latch is in said insertion position said short axis of said body is substantially aligned with said long axis of said latch, thereby providing said visual and tactile indication of the position of said latch with respect to said body and leverage to facilitate said rotatable movement of said latch with respect to said body.
